(19) Europäisches Patentamt
European Patent Office
Office européen des brevets

(11) Veröffentlichungsnummer: **0 520 290 A2**

(12) # EUROPÄISCHE PATENTANMELDUNG

(21) Anmeldenummer: **92110117.6**

(22) Anmeldetag: **16.06.92**

(51) Int. Cl.⁵: **C07C 49/255**, C07C 45/67, C07C 69/738, C11B 9/00, A61K 7/46

(30) Priorität: **28.06.91 DE 4121364**

(43) Veröffentlichungstag der Anmeldung: **30.12.92 Patentblatt 92/53**

(84) Benannte Vertragsstaaten: **CH DE FR GB IT LI NL**

(71) Anmelder: **HAARMANN & REIMER GMBH**
**Postfach 138**
**W-3450 Holzminden(DE)**

(72) Erfinder: **Hopp, Rudolf, Dr.**
**Auf dem Gehrenkamp 28**
**W-3450 Holzminden(DE)**
Erfinder: **Thielmann, Thomas, Dr.**
**Bruder-Grimm-Weg 19**
**W-3450 Holzminden(DE)**
Erfinder: **Göttsch, Wilhelm**
**Eichenhang 12**
**W-3454 Bevern(DE)**

(74) Vertreter: **Petrovicki, Wolfgang, Dr.**
**Bayer AG Konzernverwaltung RP Patente Konzern**
**W-5090 Leverkusen 1 Bayerwerk(DE)**

(54) **Ether von 1-Hydroxy-hex-5-en-2-on, Verfahren zu ihrer Herstellung und ihre Verwendung als Riechstoffe.**

(57) Ether von 1-Hydroxy-hex-5-en-2-on stellen wertvolle Riechstoffe dar, die ein sehr komplexes Duftbild besitzen und eine interessante geruchliche Variierung von Parfumölen gestatten.

EP 0 520 290 A2

Gegenstand der Erfindung sind Verbindungen der Formel

$$RO-CH_2-\underset{\underset{O}{\|}}{C}-CH_2-CH_2-CH=CH_2 \qquad (I)$$

worin

R gegebenenfalls substituiertes Alkyl, gegebenenfalls substituiertes Cycloalkyl, gegebenenfalls substituiertes Aralkyl oder gegebenenfalls substituiertes Aryl

bedeutet, Verfahren zur Herstellung dieser Verbindungen aus Ethern von 4-Hydroxy-acetessigsäureestern durch Alkenylierung mit Allylhalogenid und Verseifung und Decarboxylierung der Reaktionsprodukte.

Der Begriff "Alkyl" steht für geradkettiges oder verzweigtes Alkyl mit vorzugsweise 1 bis 12 C-Atomen, wie Methyl, Ethyl, n- und i-Propyl, n-, sek.-, i- und tert.-Butyl, n-, i- und tert.-Pentyl, n-Hexyl, i-Octyl, i-Nonyl, n-Decyl und n-Dodecyl. Diese Alkylgruppen können durch 1 bis 3 Halogenatome, vorzugsweise Chlor und/oder Fluor, oder durch eine $C_1$-$C_6$-Alkoxycarbonylgruppe substituiert sein. Substituierte Alkylgruppen umfassen demnach beispielsweise Mono-, Di- und Trifluormethyl, Monochlordifluormethyl, Methoxy- und Ethoxycarbonylmethyl.

Der Begriff "Cycloalkyl" umfaßt Cycloalkyl mit vorzugsweise 3 bis 7 C-Atomen, insbesondere mit 5 oder 6 C-Atomen, wie unsubstituiertes oder substituiertes Cyclopropyl, Cyclobutyl, Cyclopentyl, Cyclohexyl und Cycloheptyl.

"Aralkyl" enthält vorzugsweise 1 bis 6, insbesondere 1 bis 4 C-Atome im geradkettigen oder verzweigten Alkylteil und vorzugsweise 6 bis 12 C-Atome, insbesondere Phenyl oder Naphthyl als Arylteil. Beispiele für solche Aralkylgruppen umfassen Benzyl, $\alpha$-Methylbenzyl, $\alpha$,$\alpha$-Dimethylbenzyl, 2-Phenethyl, $\alpha$- und $\beta$-Naphthylmethyl. Diese Aralkylreste können 1 bis 3 Substituenten aus der Reihe Halogen (insbesondere Chlor und/oder Fluor), Nitro, Cyano, gegebenenfalls halogeniertes $C_1$-$C_4$-Alkyl oder -Alkoxy, wie beispielsweise Methyl, Ethyl, Trifluormethyl, Difluorchlormethyl, Difluormethyl, Trichlormethyl, Methoxy, Ethoxy, Trifluormethoxy, Difluorchlormethoxy und Difluormethoxy, gegebenenfalls halogeniertes $C_1$-$C_4$-Alkylmercapto, wie beispielsweise Methylmercapto, Trifluormethylmercapto, Difluorchlormethylmercapto tragen.

Der Begriff "Aryl" umfaßt unsubstituiertes oder substituiertes Aryl mit vorzugsweise 6 bis 10 Kohlenstoffatomen im Arylteil. Beispielhaft und vorzugsweise seien unsubstituiertes oder substituiertes Phenyl oder Naphthyl, insbesondere Phenyl genannt.

Für substituierte Arylreste geeignete Substituenten umfassen beispielweise Halogen, Cyano, Nitro, Hydroxy, Amino, Mercapto, geradkettiges oder verzweigtes Alkyl mit 1 bis 6 Kohlenstoffatomen, geradkettiges oder verzweigtes Halogenalkyl mit 1 bis 6 Kohlenstoffatomen und 1 bis 13 gleichen oder verschiedenen Halogenatomen, jeweils geradkettiges oder verzweigtes Alkoxy oder Alkylthio mit jeweils 1 bis 6 Kohlenstoffatomen, jeweils geradkettiges oder verzweigtes Halogenalkylthio oder Halogenalkoxy mit jeweils 1 bis 6 Kohlenstoffatomen und jeweils 1 bis 13 gleichen oder verschiedenen Halogenatomen, jeweils geradkettiges oder verzweigtes Alkenyl oder Alkinyl mit 2 bis 6 Kohlenstoffatomen, jeweils geradkettiges oder verzweigtes Alkenyloxy oder Alkinyloxy mit 2 bis 6 Kohlenstoffatomen, Cycloalkyl mit 3 bis 8 Kohlenstoffatomen, Phenyl, Phenoxy, Phenylthio, Phenylalkylthio oder Phenylalkyloxy mit jeweils 1 bis 4 Kohlenstoffatomen im geradkettigen oder verzweigten Alkylteil.

Die erfindungsgemäßen Verbindungen I können nach folgendem Schema hergestellt werden, wobei X für Halogen (z.B. Brom, Chlor oder Iod) steht, R die oben angegebene Bedeutung besitzt und R' unabhängig von R die oben für R angegebenen Bedeutungen annehmen kann.

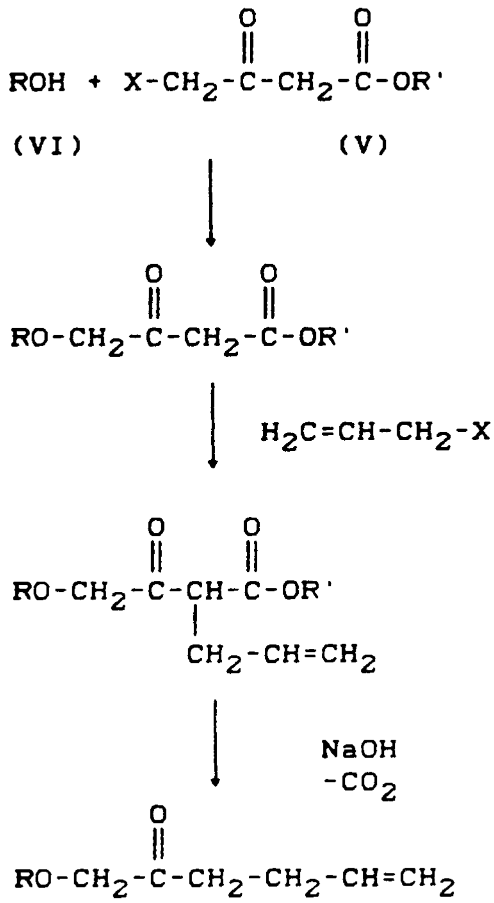


(VI) (V)

(III)

(IV)

(II)

(I)

Die erfindungsgemäßen als Riechstoffe verwendbaren Verbindungen (I) sind durch Verseifung und Decarboxylierung von 4-Organoxy-α-ally-acetessigsäureestern (II) zugänglich, die aus den entsprechenden 4-Organoxy-acetessigsäureestern (III) und Allylhalogenid (IV) hergestellt werden können Die Verbindungen (III) wiederum sind mindestens teilweise bekannt und können aus 4-Halogenacetessigsäureestern (V) und Alkoholen bzw. Phenolen (VI) in Gegenwart starker Basen oder aus (V) und Alkoholaten bzw. Phenolaten, wie es beispielsweise bei T. Kato et al., J. Chem. Soc., Perkin I (1979), 529 beschrieben ist, oder durch analoge Verfahren hergestellt werden.

Im einzelnen kann man die dazu zu verwendenden Alkoholate bzw. Phenolate aus den Alkoholen bzw. Phenolen (VI), wie üblich, durch Umsetzung mit Alkalimetallen, vorzugsweise Lithium, Natrium oder Kalium, herstellen. Dabei und für die weitere Reaktion können die Alkohole bzw. Phenole (VI) als Lösungsmittel dienen. Wie erwähnt, kann man die Umsetzung der 4-Halogen-acetessigsäureester (V) auch in Gegenwart starker Basen, wie z.B. Alkalimetallamide, Alkalimetallhydride oder komplexer Hydride wie Natriumborhydrid, geeigneter Alkoholate oder Amine vornehmen.

Als Halogen in den Verbindungen (V) können auch Brom oder Iod dienen; Chlor ist aber schon allein aus Kostengründen bevorzugt.

Das Molverhältnis (V)/(VI) beträgt in der Regel 1 : 1,5 bis 1 : 0,1, vorzugsweise 1 : 1,1 bis 1 : 0,5. Die Anwesenheit organischer dipolarer aprotischer Lösungsmittel, wie Dimethylsulfoxid und vorzugsweise Dimethylformamid, kann für die Reaktion von Vorteil sein. Die Reaktion kann bei Temperaturen von 0 bis 150°C, vorzugsweise 20 bis 80°C durchgeführt werden. Man wird in der Regel abwarten, bis der Umsatz quantitativ ist, und dann wie üblich aufarbeiten, d.h. das organische Lösungsmittel abziehen, mit wäßriger Mineralsäure neutralisieren oder sauerstellen, das Reaktionsprodukt mit organischem Lösungsmittel extrahieren, das Lösungsmittel abziehen und den Rückstand gegebenenfalls, z.B. durch Destillation, weiter reinigen.

Für die Umsetzung des so erhaltenen 4-Organoxy-acetessigsäureesters (III) mit dem Allylhalogenid (IV) wählt man im allgemeinen Molverhältnisse (IV)/(III) von 3 : 1 bis 0,5 : 1, vorzugsweise 1,2 : 1 bis 0,9 : 1. Da die Reaktion exotherm verläuft, empfiehlt es sich, zunächst zu kühlen, so daß die Temperatur des Reaktionsgemisches 200°C, vorzugsweise 60°C, möglichst nicht überschreitet. Zur quantitativen Umsetzung sollte das Reaktionsgemisch nach beendeter Zugabe von (IV) noch 0,5-24 Stunden, vorzugsweise 1-5 Stunden bei einer Temperatur von 30 bis 200°C, vorzugsweise 50 bis 60°C, belassen werden. Die Reaktion

kann in Gegenwart der oben für die Reaktion zwischen den Verbindungen (V) und (VI) erwähnten Basen und Lösungsmittel durchgeführt werden. Das Reaktionsgemisch kann wie bei der Synthese des 4-Organoxy-acetessigsäureesters (III) geschildert aufgearbeitet werden.

Die Verseifung und Decarboxylierung des erhaltenen $\alpha$-Allyl-acetessigsäureesters (II) erfolgt am besten in wäßriger Natron- oder Kalilauge bei Temperaturen zwischen 70 und 160, vorzugsweise 90 und 140$^0$C. Die Menge Lauge wird so berechnet, daß pro Mol Ester (III) mindestens 1 Mol, vorzugsweise aber 1,5 bis 5 Mol, Alkalihydroxid anwesend sind. Zur besseren Vermischbarkeit kann ein wassermischbarer Alkohol, wie z.B. Ethanol, mitverwendet werden. Die Decarboxylierung setzt bei den obengenannten Temperaturen spontan in dem Maß ein, wie die Verseifung fortgeschritten ist. Das gewünschte Reaktionsprodukt (I) kann mit organischem Lösungsmittel extrahiert und aus diesem durch Abziehen des Lösungsmittels gewonnen werden. Zur Reinigung kann sich eine Destillation anschließen.

Weiterer Gegenstand der Erfindung sind also Verbindungen der Formel (II)

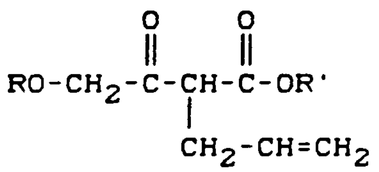

(II)

worin R und - unabhängig von R - R' die oben unter R genannte Bedeutung besitzen.

Weiterer Gegenstand der Erfindung ist ein Verfahren zur Herstellung der Verbindungen (II) durch Umsetzung der Verbindungen (III) mit Allylhalogenid, und schließlich ist Gegenstand der Erfindung ein Verfahren zur Herstellung der Verbindungen (I) durch Verseifung und Decarboxylierung der Verbindungen (II).

Die erfindungsgemäßen Verbindungen (I) sind Riechstoffe, die ein sehr komplexes Duftbild besitzen und eine interessante geruchliche Variierung von Parfumölen gestatten. Da die Verbindungen (I) außerdem sehr duftstark sind, lassen sich bereits mit kleinen Zusatzmengen gute Effekte erzielen.

So lauten einige beispielmäßige Geruchsbeschreibungen:

1-Cyclohexyloxy-hex-5-en-2-on:

Riechstoff mit einer sehr fruchtigen Galbanumnote. Die Hauptnoten erinnern stark an Galbanumöl und Ananas. Daneben finden sich holzige und krautige Noten, die an Kamille und Stroh erinnern. Die Ananasnote wird begleitet von feigen-, rum- und arrakartigen Nuancen. Daneben findet sich eine leicht animalische zibethartige Note, - Dieser durftstarke Riechstoff vereint in sich Duftnoten, die sonst nur durch Mischung mehrerer Riechstoffe zu erreichen sind. -

1-(3-Methylcyclohexyloxy)-hex-5-en-2-on:

Duftstarker und haftfester Riechstoff mit krautig grünen Nuancen, die vorwiegend an Kamille, Baldrian, Basilikum und Galbanum erinnern. Die fruchtigen Nuancen erinnern an den Duft von getrockneten Früchten und Ananas. Daneben sind blumige, veilchenähnliche Elemente zu erkennen.

Die erfindungsgemäßen Verbindungen (I) können in Kombination mit anderen, an sich bekannten Riechstoffen (Arctander, Perfume and Flavor Chemicals, Montclair, N.J. (USA), 1969) und etherischen Ölen (Arctander, Perfume and Flavor Materials of natural Origin, Elisabeth, N.J. (USA), 1960) angewendet werden und führen zu Parfumbasen und Riechstoffkombinationen mit ausdruckstarken Noten, die sich hervorragend für die Parfumierung von Fertigprodukten des Aerosol-, Waschmittel- und des chemischtechnischen-Sektors, insbesondere aber des Feinparfumerie-und Kosmetiksektors eignen, z.B. für Detergentien, Haarpflegemittel, Schaumbäder, Badesalz, Geschirrspülmittel, Waschpulver, Seifen, Antiperspirans, Puder, Cremes, Rasierwasser, After-Shave-Lotions, Raumluftverbesserer, WC-Reiniger, Raum-Sprays, Antiperspirans-Sprays, Deodorant-Sprays, Körper-Sprays, Sonnenschutzmittel.

Die Zusatzmenge zu Parfumölen beträgt zwischen 0,1 bis 10 Gew.-%, vorzugsweise jedoch zwischen 0,5 bis 3 Gew.-%, bezogen auf das Parfumöl (vor dem Zusatz). Die Herstellung der Parfumkompositionen und parfumierten Produkte kann auf übliche Weise, beispielsweise durch Zusammengeben der Komponenten, erfolgen.

Die Prozentangaben der nachfolgenden Beispiele beziehen sich, falls nicht anders angegeben, auf das Gewicht.

## Beispiele

### Beispiel 1

a) In einem Dreihalskolben, der mit Rührer, Tropftrichter, Trockenrohr und Rückflußkühler ausgestattet ist, werden 320 g 4-Cyclohexyloxy-acetessigsäureethylester (1,4 Mol) vorgelegt und mit einer aus 32 g Natrium (1,4 Mol) und 475 g Ethanol hergestellten Na-ethanolat-Lösung bei 50°C innerhalb von 30 Minuten versetzt. Dann werden unter Kühlung bei 50 - 60°C innerhalb von 30 Minuten 170 g Allylbromid (1,4 Mol) zudosiert. Es wird 2 Stunden bei 50°C nachgerührt, anschließend Ethanol abdestilliert und das verbleibende Reaktionsgemisch mit Salzsäure auf pH = 3 eingestellt. Nach Trennung der Phasen wird die wäßrige Phase zweimal mit je 750 ml Toluol extrahiert, die organischen Phasen vereinigt, eingeengt und destilliert. Man erhält 2-(Cyclohexyloxyacetyl)-4-pentensäureethylester. (T(Sumpf) = 152-179°C, T(Kopf) = 102-127°C; 1 mbar).

b) 140 g 2-(Cyclohexyloxyacetyl)-4-pentensäureethylester werden in 650 g 5 %iger wäßriger Natronlauge 4 Stunden unter Rückfluß erhitzt. Das Reaktionsgemisch wird mit 500 ml Ether versetzt. Die organische Phase wird abgetrennt, eingeengt und destilliert. Man erhält ca. 60 g 1-Cyclohexyloxy-hex-5-en-2-on. (T-(Sumpf = 115-155°C, T(Kopf) = 83°C; 0,8 mbar).

### Beispiel 2

Analog Beispiel 1 wurde 1-(3-Methyl-cyclohexyloxy)-hex-5-en-2-on hergestellt; Kp. 103°C/2 mbar.

## Anwendung

Eine Riechstoffkomposition mit blütig grünem Durft wird durch Mischen folgender Bestandteile hergestellt (Mengenangabe in Gramm):

| | |
|---|---|
| Hexenylsalicylat | 80 |
| Orangenöl kaltgepreßt | 30 |
| Muguet 10689 F (Maiglöckchen synth.) | 100 |
| Jasmin 10400 (Jasmin synth.) | 100 |
| Bergamotteöl synthessence | 100 |
| Dimethylbenzylcarbinylacetat | 30 |
| Iraldein Gamma | 80 |
| Linalylacetat | 40 |
| Cumarin | 30 |
| Moschus Keton | 60 |
| Phenylethylalkohol | 80 |
| Isoeugenol | 4 |
| Eugenol | 6 |
| p-tert.-Butylcyclohexylacetat | 20 |
| o-tert.-Butylcyclohexylacetat | 20 |
| Ethylvanillin | 2 |
| Cyclopentadecanolid 50 % | 30 |
| Hydroxybenzylaceton-para | 1 |
| Undecylenaldehyd | 1 |
| Hexenylacetat | 2 |
| Citraldiethylacetal | 10 |
| Evernyl | 4 |
| Galbanum synthresin | 5 |
| Petitgrainöl Paraguay | 15 |
| Diethylphthlat | 140 |
| 1-Cyclohexyloxy-hex-5-en-2-on | 10 |
| | 1 000 |

Der Zusatz von 1 % 1-Cyclohexyloxy-hex-en-2-on verändert das Duftbild so, daß eine wertvoller anmutende Komposition entsteht, wie sie sonst nur durch den Zusatz sehr teurer Naturblüten oder

Balsamextrakte zu erreichen ist. So wird das beschriebene Beispiel durch Zusatz von 1 % des obigen Ketons mit einer süß-fruchtigen Note abgerundet, die eine Verbindung zu blütigen Chypre- und Orientdüften schafft und die geruchliche Akzeptanz eindeutig verbessert.

## Patentansprüche

1. Verbindungen der Formel

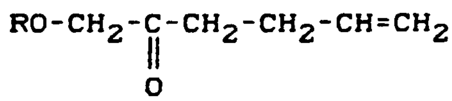

(I)

worin
R gegebenenfalls substituiertes Alkyl,
gegebenenfalls substituiertes Cycloalkyl,
gegebenenfalls substituiertes Aralkyl oder
gegebenenfalls substituiertes Aryl
bedeutet.

2. Verbindungen nach Anspruch 1, worin
R gegebenenfalls substituiertes $C_1$-$C_{12}$-Alkyl, gegebenenfalls substituiertes $C_3$-$C_7$-Cycloalkyl, gegebenenfalls substituiertes Aralkyl mit 1 bis 6 C-Atomen im Alkyl- und 6 bis 12 C-Atomen im Arylteil oder gegebenenfalls substituiertes $C_6$-$C_{10}$-Aryl
bedeutet.

3. Verbindungen nach Anspruch 1, worin
R Cyclohexyl oder Methylcyclohexyl
bedeutet.

4. Verbindungen der Formel

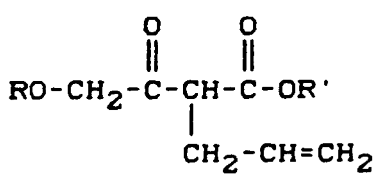

(II)

worin
R und - unabhängig von R -
R' die Bedeutung von R in Anspruch 1 besitzen.

5. Verfahren zur Herstellung der Verbindungen nach Anspruch 4 durch Umsetzung von Verbindungen der Formel

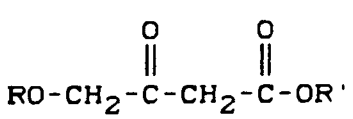

(III)

worin
R und - unabhängig von R -
R' die Bedeutung von R in Anspruch 1 besitzen,
mit Allylhalogenid.

6. Verfahren zur Herstellung der Verbindungen nach Anspruch 1 durch Verseifung und Decarboxylierung von Verbindungen nach Anspruch 4.

7. Verwendung der Verbindungen nach Anspruch 1 als Riechstoffe.